# EUROPÄISCHE PATENTSCHRIFT

(11) **EP 0 704 431 B1**
(45) Veröffentlichungstag und Bekanntmachung des Hinweises auf die Patenterteilung: **24.05.2000**
(21) Anmeldenummer: 95112998.0
(22) Anmeldetag: 18.08.1995
(51) Int. Cl.: C07C 317/44, C07C 317/46, C07C 317/48, C07C 311/16, C07C 311/29, A61K 31/155, A61K 31/18, A61K 31/275

(54) **Ortho-substituierte Benzoesäurederivate**
Ortho-substituted benzoic acid derivatives
Dérivés d'acide benzoique ortho-substitués

(30) Priorität: 28.08.1994 DE 4430212
(43) Veröffentlichungstag der Anmeldung: 03.04.1996
(73) Patentinhaber: MERCK PATENT GmbH, 64293 Darmstadt (DE)
(72) Erfinder: Baumgarth, Manfred, Dr., D-64297 Darmstadt (DE); Gericke, Rolf, Dr., D-64342 Seeheim (DE); Dorsch, Dieter, Dr., D-64372 Ober-Ramstadt (DE); Minck, Klaus-Otto, Dr., D-64372 Ober-Ramstadt (DE); Beier, Norbert, Dr., D-64354 Reinheim (DE)

(56) Entgegenhaltungen:
- EP-A- 0 416 499
- EP-A- 0 556 673
- EP-A- 0 556 674
- EP-A- 0 589 336
- EP-A- 0 667 341
- EP-A- 0 690 048
- DATABASE REGISTRY CHEMICAL ABSTRACTS SERVICE, COLUMBUS, OHIO, US (STN), XP002018384 & DATABASE CHEMABS CHEMICAL ABSTRACTS SERVICE, COLUMBUS, OHIO, US

## Beschreibung

Die Erfindung betrifft ortho-substituierte Benzoesäurederivate der Formel I 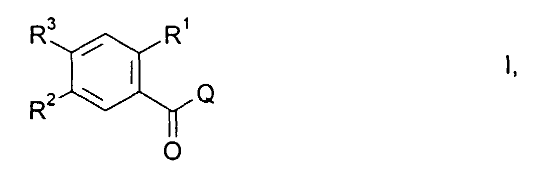 worin
- R¹: A,
- R²: -SO₂-R⁶,
- R³: CN oder Hal
- Q: -N=C(NH₂)₂,
- R⁶: A,
- A: Alkyl mit 1 bis 6 C-Atomen
bedeuten,
sowie deren pharmazeutisch verträglichen Salze.

Der Erfindung lag die Aufgabe zugrunde, neue Verbindungen mit wertvollen Eigenschaften aufzufinden, insbesondere solche, die zur Herstellung von Arzneimitteln oder als Zwischenprodukte zur Herstellung anderer Wirkstoffe verwendet werden können.

Es wurde gefunden, daß die Verbindungen der Formel I, insbesondere diejenigen in denen Q -N=C(NH₂)₂ bedeutet, und ihre physiologisch unbedenklichen Salze bei guter Verträglichkeit wertvolle pharmakologische Eigenschaften besitzen.

Ferner eignen sich die Substanzen der Formel I besonders als Zwischenprodukte, die insbesondere zur Synthese von anderen, hier nicht beanspruchten Hemmstoffen des zellulären Na⁺/H⁺-Antiporters vom Acylguanidintyp verwendet werden können.

Bei den neuen Verbindungen handelt es sich um Inhibitoren des zellulären Na⁺/H⁺-Antiporters, d.h. um Wirkstoffe, die den Na⁺/H⁺-Austauschmechanismus der Zellen hemmen (Düsing et al., Med. Klin. 87, 378-384 (1992)) und die somit gute Antiarrhythmika darstellen, die sich insbesondere zur Behandlung von Arrhythmien eignen, die als Folge von Sauerstoffmangel auftreten.

Der bekannteste Wirkstoff der Gruppe der Acylguanidine ist Amilorid. Diese Substanz zeigt jedoch in erster Linie eine blutdrucksenkende und saluretische Wirkung, was insbesondere bei der Behandlung von Herz-Rhythmus-Störungen unerwünscht ist, während die antiarrhythmischen Eigenschaften nur sehr schwach ausgeprägt sind.

Man kennt strukturell ähnliche Verbindungen beispielsweise aus EP 04 16 499.

Andere ortho-substituierte Benzoylguanidine mit antiarrythmischer Wirkung sind z.B. bekannt aus der EP 0 556 673, wohingegen Benzoylguanidine, die keine ortho-Substitution aufweisen, z.B. in EP 0 416 499, EP 0 589 336 oder EP 0 556 674 beschrieben sind.

Die erfindungsgemäßen Substanzen vom Acylguanidintyp der vorliegenden Anmeldung zeigen eine gute kardioprotektive Wirkung und eignen sich daher besonders zur Infarktbehandlung, Infarktprophylaxe und zur Behandlung von Angina pectoris. Ferner wirken die Substanzen allen pathologischen hypoxischen und ischämischen Schädigungen entgegen, so daß die dadurch primär oder sekundär verursachten Krankheiten behandelt werden können. Die Wirkstoffe sind ebenfalls für präventive Anwendungen gut geeignet.

Aufgrund der protektiven Wirkungen dieser Substanzen bei pathologischen hypoxischen oder ischämischen Situationen resultieren daraus weitere Anwendungsmöglichkeiten bei chirurgischen Eingriffen zum Schutz zeitweilig minderversorgter Organe, bei Organtransplantationen zum Schutz der entnommenen Organe, bei angioplastischen Gefäß- oder Herzeingriffen, bei Ischämien des Nervensystems, bei der Therapie von Schockzuständen und zur präventiven Verhinderung der essentiellen Hypertonie.

Ferner können die Verbindungen auch als Therapeutika bei durch Zellproliferation bedingten Erkrankungen wie Arteriosklerose, diabetische Spätkomplikationen, Tumorerkrankungen, fibrotischen Erkrankungen, insbesondere von Lunge, Leber und Nieren sowie Organhypertrophien und -hyperplasien, eingesetzt werden. Darüber hinaus eignen sich die Substanzen zur diagnostischen Anwendung zur Erkennung von Krankheiten, die von einer gesteigerten Aktivität des Na⁺/H⁺-Antiporters z.B. in Erythrozyten, Thrombozyten oder Leukozyten begleitet werden.

Die Wirkungen der Verbindungen können mit Hilfe an sich bekannter Methoden ermittelt werden, wie sie z.B. von N. Escobales and J. Figueroa in J. Membrane Biol. 120, 41-49 (1991) oder von L. Counillon, W. Scholz, H.J. Lang und J. Pouysségur in Mol. Pharmacol. 44, 1041-1045 (1993) angegeben werden.

Als Versuchstiere eignen sich z.B. Mäuse, Ratten, Meerschweinchen, Hunde, Katzen, Affen oder Schweine.

Die Verbindungen der Formel I, insbesondere die Acylguanidin-Derivate, können daher als Arzneimittelwirkstoffe in der Human- und Veterinärmedizin verwendet werden. Ferner können sie als Zwischenprodukte zur Herstellung weiterer Arzneimittelwirkstoffe Verwendung finden, insbesondere für solche, die den zellulären Na⁺/H⁺-Antiport hemmen.

Gegenstand der Erfindung sind somit ortho-substituierte Benzoesäurederivate der Formel I sowie ihre physiologisch unbedenklichen Salze.

In den angegebenen Formeln bedeutet A eine verzweigte oder unverzweigte Alkylgruppe mit 1-6, bevorzugt 1-4, insbesondere 1, 2 oder 3 C-Atomen, im einzelnen vorzugsweise Methyl und Ethyl, ferner bevorzugt Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

R¹ bedeutet A, wobei A insbesondere die zuvor angegebenen besonders bevorzugten Bedeutungen hat.

R² bedeutet SO₂A.

R³ bedeutet vorzugsweise Halogen wie F, insbesondere aber Br oder Cl, ferner aber auch CN.

R⁶ steht für A, insbesondere für Methyl.

Q bedeutet -N=C(NH₂)₂.

Gegenstand der Erfindung ist ferner ein Verfahren zur Herstellung von ortho-substituierten Benzoesäurederivaten der oben angegebenen Formel I sowie von deren Salzen, dadurch gekennzeichnet, daß man
- eine Carbonsäure der Formel I (Q = Hydroxy), in ein Acylguanidin überführt,
und/oder daß man
- eine Verbindung der Formel I durch Behandeln mit einer Säure oder einer Base in eines ihrer Salze überführt.

Die Verbindungen der Formel I werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; Organic Reactions, John Wiley & Sons, Inc., New York; sowie in der oben angegebenen Patentanmeldung) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Verbindungen der Formel I können ferner durch elektrophile Substitutionsreaktionen am Aromaten hergestellt werden, sofern andere Nebenreaktionen ausgeschlossen werden können. Sie können z.B. unter den Bedingungen der Friedel-Crafts-Reaktionen chloriert, bromiert, alkyliert oder acyliert werden, indem man das entsprechende Halogen oder Alkylchlorid bzw. Alkylbromid unter Katalyse von Lewis-Säuren, wie z.B. AlCl₃, FeBr₃ oder Fe, oder das gewünschte Acylchlorid in Gegenwart einer Lewis-Säure bei Temperaturen zwischen 30° und 150°, zweckmäßig zwischen 50° und 150° in einem inerten Lösungsmittel, wie z.B. Kohlenwasserstoffen, THF oder Tetrachlorkohlenstoff, mit der zu derivatisierenden Verbindung der Formel I umsetzt.

Die Verbindungen der Formel I können auch erhalten werden, indem man sie aus ihren funktionellen Derivaten durch Solvolyse, insbesondere Hydrolyse, oder durch Hydrogenolyse in Freiheit setzt.

Besonders bevorzugte Ausgangsstoffe für die Hydrolyse sind Verbindungen der Formel I, die über eine Nitrilgruppe verfügen. Derartige Verbindungen können unter an sich bekannten Reaktionsbedingungen, z.B. in schwefelsaurem Medium, über das entsprechende Carbonsäureamidderivat, oder allgemein durch Erhitzen mit starken Säuren oder Basen zur Carbonsäure hydrolysiert werden.

Beispielsweise ist es möglich, daß man einen Ester der Formel I (Q = OA) verseift, zweckmäßig durch Solvolyse nach einer an sich bekannten Methode, z.B. mit NaOH oder KOH in Dioxan/Wasser bei Temperaturen zwischen 0 und 40°, vorzugsweise 10 und 30°.

Zur Veresterung kann man eine Säure der Formel I mit einem Überschuß eines Alkohols behandeln, zweckmäßig in Gegenwart einer starken Säure wie Salzsäure oder Schwefelsäure bei Temperaturen zwischen 0 und 100, vorzugsweise 20 und 50°.

Ferner kann eine geeignete Verbindung der Formel I, in der Q ungleich Guanidinyl ist, durch Umsetzung mit Guanidin in ein Acylguanidin überführt werden.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Trifluoressigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Die freien Basen der Formel I können, falls gewünscht, aus ihren Salzen beispielsweise durch Behandlung mit starken Basen wie Natrium- oder Kaliumhydroxid, Natrium- oder Kaliumcarbonat in Freiheit gesetzt werden.

Die Verbindungen der Formel I und ihre physiologisch unbedenklichen Salze können zur Herstellung pharmazeutischer Zubereitungen verwendet werden, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I, in der Q Guanidinyl bedeutet und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlenhydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z.B. zur Herstellung von Injektionspräparaten verwendet werden.

Insbesondere für die topische Anwendung kommen auch liposomale Zubereitungen in Betracht. Die angegebenen Zubereitungen können sterilisiert sein und/oder Hilfsstoffe wie Geleit-, Konservierungs-, Stabilisierungs- und/oder Netzmittel, Emulgatoren, Salze zur Beeinflussung des osmotisches Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems. Sie eignen sich daher zur Behandlung von Arrhythmien, insbesondere wenn diese durch Sauerstoffmangel hervorgerufen werden, von Angina pectoris, Infarkten, Ischämien des Nervensystems wie z.B. Schlaganfall oder Hirnödeme, von Schockzuständen und zur Präventiv-behandlung.

Die Substanzen können ferner als Therapeutika bei Erkrankungen eingesetzt werden, bei denen Zellproliferationen eine Rolle spielen wie Arteriosklerose, diabetische Spätkomplikationen, Tumorerkrankungen, Fibrosen sowie Organhypertrophien und -hyperplasien.

Dabei werden die erfindungsgemäßen Substanzen in der Regel in Analogie zu bekannten Antiarrhythmika, z.B. Aprindin, verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet "übliche Aufarbeitung":

Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

### Beispiel 1

Man rührt eine Lösung von 200 g 4-Chlor-2-methyl-benzoesäure und 410 g Chlorsulfonsäure 6 Std. bei 140° und gießt die Reaktionsmischung anschließend auf Eis. Der Niederschlag wird abgesaugt und portionsweise in eine Suspension von 447 g Natriumsulfit in 1170 ml Wasser bei 10° eingetragen, wobei gleichzeitig Natronlauge zugegeben wird, so daß ein pH-Wert von 9 erreicht wird. Man rührt noch 3 Std. nach und säuert anschließend unter Eiskühlung an. Der entstandene Niederschlag wird wiederum abgesaugt und zusammen mit 610 g Methyliodid in eine Mischung aus 575 ml Methanol und 350 ml Wasser eingebracht. Man stellt einen pH-Wert von 9 ein und kocht 36 Std., bevor man das Lösungsmittel entfernt und wie üblich aufarbeitet. Man erhält den 2-Methyl-4-chlor-5-methylsulfonyl-benzoesäuremethylester, F. 151°.

Analog erhält man durch Umsetzung mit Chlorsulfonsäure, Reduktion und Methylierung
aus 2-Ethyl-4-chlor-benzoesäure den
   2-Ethyl-4-chlor-5-methylsulfonyl-benzoesäuremethylester, F. 98-100°;
aus 2-Methyl-4-brom-benzoesäure den
   2-Methyl-4-brom-5-methylsulfonyl-benzoesäuremethylester, F. 150°;
aus 2-Brom-4-methyl-benzoesäure den
   2-Brom-4-methyl-5-methylsulfonyl-benzoesäuremethylester, F. 160-165°;
aus 2-Ethyl-4-brom-benzoesäure den
   2-Ethyl-4-brom-5-methylsulfonyl-benzoesäuremethylester;
aus 2-Ethyl-4-chlor-benzoesäure den
   2-Ethyl-4-chlor-5-methylsulfonyl-benzoesäuremethylester;
aus 2-Methyl-4-fluor-benzoesäure den
   2-Methyl-4-fluor-5-methylsulfonyl-benzoesäuremethylester.

### Beispiel 2

10 g 2-Methyl-4-chlor-5-methylsulfonyl-benzoesäuremethylester werden unter Eiskühlung in eine Mischung aus 30 ml konz. HCI und 200 ml Methanol gegeben und 2 Std. gerührt. Der entstandene Niederschlag wird abgesaugt und aus Methanol umkristallisiert. Man erhält 2-Methyl-4-chlor-5-methylsulfonyl-benzoesäure, F. 217-218°.

Analog erhält man durch Verseifung
von 2-Ethyl-4-chlor-5-methylsulfonyl-benzoesäuremethylester:
   2-Ethyl-4-chlor-5-methylsulfonyl-benzoesäure, F. 180-183°;
von 2-Methyl-4-brom-5-methylsulfonyl-benzoesäuremethylester:
   2-Methyl-4-brom-5-methylsulfonyl-benzoesäure, F. 221-222°;
von 2-Ethyl-4-brom-5-methylsulfoyl-benzoesäurenmethylester:
   2-Ethyl-4-brom-5-methylsulfonyl-benzoesäure;
von 2-Methyl-4-fluor-5-methylsulfonyl-benzoesäuremethylester:
   2-Methyl-4-fluor-5-methylsulfonylbenzoesäure;
von 2-Ethyl-4-fluor-5-methylsulfonyl-benzoesäuremethylester:
   2-Ethyl-4-fluor-5-methylsulfonyl-benzoesäure;

### Beispiel 3

15 g 2-Ethyl-4-chlor-5-methylsulfonyl-benzoesäuremethylester werden in 100ml Methanol und 50 ml 2 n Natronlauge 1 Std. bei Raumtemperatur gerührt. Anschließend wird die Reaktionsmischung eingeengt und mit 200 ml Eiswasser versetzt. Nach Ansäuern mit konz. HCI wird der entstandene Niederschlag abgesaugt, mit Ether gewaschen und getrocknet. Man erhält 2-Ethyl-4-chlor-5-methylsulfonyl-benzoesäure, F. 180-183°.

### Beispiel 4

2 g 1-Chlor-2-methylsulfonyl-4,5-dimethylbenzol [erhältlich nach Bsp. 1 ausgehend von 1-Chlor-3,4-dimethylbenzol durch Umsetzung mit Chlorsulfonsäure, Natriumsulfit und Methyliodid] werden zusammen mit 40 ml 15-%iger Salpetersäure im Autoklaven 5 Std. bei 132° erhitzt. Anschließend wird die Reaktionsmischung mit Essigsäureethylester extrahiert und wie üblich aufgearbeitet. Man erhält 2-Methyl-4-chlor-5-methylsulfonyl-benzoesäure, F. 217-218°.

### Beispiel 5

8,3 g 2-Methyl-4-chlor-5-methylsulfonyl-benzoesäure werden mit 70 ml N-Methylpyrrolidon und 7,4 g CuCN zusammengegeben und 3 Tage bei 150° gerührt. Anschließend gießt man die Reaktionsmischung in 250 ml Wasser ein und arbeitet wie üblich auf. Nach Umkristallisieren aus Methanol erhält man 2-Methyl-4-cyan-5-methylsulfonyl-benzoesäure, F. 248-249°.

Analog erhält durch Umsetzung von CuCN
mit 2-Ethyl-4-chlor-5-methylsulfonyl-benzoesäure:
2-Ethyl-4-cyan-5-methylsulfonyl-benzoesäure;

### Beispiel 6

1,0 g 2-Methyl-4-cyan-5-methylsulfonyl-benzoesäure [erhältlich nach Bsp. 5] werden in 15 ml 1-Methylpyrrolidon gelöst, mit 0,67 g 1-Methyl-2-chlorpyridiniumchlorid versetzt und 15 Min. gerührt. Anschließend fügt man 1 Equivalent Guanidiniumchlorid sowie 2,6 ml Diisopropylethylamin hinzu und rührt 1 Std. bei Raumtemperatur. Nach üblicher Aufarbeitung erhält man nach Chromatographie über Kieselgel (Flash-Verfahren, Ethylacetat/10% Methanol) und anschließende Behandlung mit HCI N-Diaminomethylen-2-methyl-4-cyan-5-methylsulfonyl-benzamid, Hydrochlorid, F. 227-228°.

Analog erhält man durch Umsetzung von Guanidinumchlorid
mit 2-Ethyl-4-cyan-5-methylsulfonyl-benzoesäure:
N-Diaminomethylen-2-ethyl-4-cyan-5-methylsulfonyl-benzamid, Hydrochlorid.

### Beispiel 7

Man kocht eine Lösung von 1,8 g 2-Methyl-4-brom-5-methylsulfonylbenzoesäuremethylester [erhältlich nach Bsp. 1] und 1,5 g Guanidin in 50 ml Methanol fünf Stunden und entfernt anschließend das Lösungsmittel. Der Rückstand wird mit Wasser behandelt, das verbleibende Kristallisat abgesaugt und mit verd. Natronlauge behandelt. Man filtriert den festen Rückstand ab, kristallisiert aus Ethanol um und erhält N-Diaminomethylen-2-methyl-4-brom-5-methylsulfonyl-benzamid, F. 207-208°.

Analog erhält man durch Umsetzung von Guanidin
mit 2-Methyl-4-chlor-5-methylsulfonyl-benzoesäuremethylester:
   N-Diaminomethylen-2-methyl-4-chlor-5-methylsulfonyl-benzamid, F. 204-205°;
mit 2-Ethyl-4-chlor-5-methylsulfonyl-benzoesäuremethylester:
   N-Diaminomethylen-2-ethyl-4-chlor-5-methylsulfonyl-benzamid, F. 160-162°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann beispielsweise in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Ortho-substituierte Benzoesäurederivate der Formel I 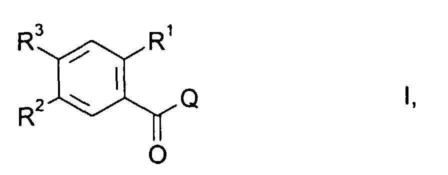 worin
R¹ A,
R² -SO₂-R⁶,
R³ CN oder Hal,
Q -N=C(NH₂)₂,
R⁶ A,
A Alkyl mit 1 bis 6 C-Atomen
bedeuten,
sowie deren pharmazeutisch verträglichen Salze.

2. (a) N-Diaminomethylen-2-methyl-4-brom-5-methylsulfonylbenzamid;
(b) N-Diaminomethylen-2-methyl-4-chlor-5-methylsulfonylbenzamid;
(c) N-Diaminomethylen-2-methyl-4-cyan-5-methylsulfonylbenzamid;
(d) N-Diaminomethylen-2-ethyl-4-brom-5-methylsulfonylbenzamid;
(e) N-Diaminomethylen-2-ethyl-4-chlor-5-methylsulfonylbenzamid;
(f) N-Diaminomethylen-2-methyl-4-fluor-5-methylsulfonylbenzamid;
(g) N-Diaminomethylen-2-ethyl-4-fluor-5-methylsulfonyl-benzamid;
gemäß Anspruch 1, sowie deren physiologisch unbedenkliche Salze.

3. Verfahren zur Herstellung von ortho-substituierten Benzoesäurederivaten der Formel I gemäß Anspruch 1 sowie von deren Salzen, dadurch gekennzeichnet, daß man
- eine Carbonsäure der Formel I (Q = Hydroxy) in ein Acylguanidin überführt,
und/oder daß man
- eine Verbindung der Formel I durch Behandeln mit einer Säure oder einer Base in eines ihrer Salze überführt.

4. Verfahren zur Herstellung einer pharmazeutischen Zubereitung, dadurch gekennzeichnet, daß man eine Verbindung der Formel I gemäß Anspruch 1 in der Q -N=C(NH₂)₂ bedeutet und/oder eines ihrer physiologisch unbedenklichen Salze zusammen mit mindestens einem festen, flüssigen oder halbflüssigen Träger- oder Hilfsstoff in eine geeignete Darreichungsform bringt.

5. Pharmazeutische Zubereitung, gekennzeichnet durch einen Gehalt an mindestens einer Verbindung der Formel I, gemäß Anspruch 1, in der Q -N=C(NH₂)₂ bedeutet, und/oder einem ihrer physiologisch unbedenklichen Salze.

6. Verwendung einer Verbindung der Formel I gemäß Anspruch 1, in der Q -N=C(NH₂)₂ bedeutet, und/oder eines ihrer physiologisch unbedenklichen Salze zur Herstellung von Arzneimitteln.

7. Verwendung von Verbindungen der Formel I nach Anspruch 1 in der Q -N=C(NH₂)₂ bedeutet, zur Herstellung von Arzneimitteln zur Behandlung von Arrhythmien, Angina pectoris, Infarkten sowie zur Präventiv-Behandlung der genannten Indikationen.

## Claims

1. Ortho-substituted benzoic acid derivatives of the formula I 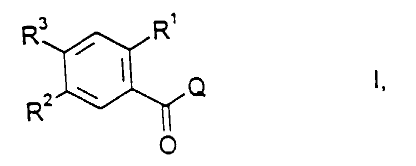 in which
R¹ is A,
R² is -SO₂-R⁶,
R³ is CN or Hal,
Q is -N=C(NH₂)₂,
R⁶ is A,
A is alkyl having 1 to 6 C atoms
and their pharmaceutically tolerable salts.

2. (a) N-Diaminomethylene-2-methyl-4-bromo-5-methylsulphonylbenzamide;
(b) N-diaminomethylene-2-methyl-4-chloro-5-methylsulphonylbenzamide;
(c) N-diaminomethylene-2-methyl-4-cyano-5-methylsulphonylbenzamide;
(d) N-diaminomethylene-2-ethyl-4-bromo-5-methylsulphonylbenzamide;
(e) N-diaminomethylene-2-ethyl-4-chloro-5-methylsulphonylbenzamide;
(f) N-diaminomethylene-2-methyl--4-fluoro-5-methylsulphonylbenzamide;
(g) N-diaminomethylene-2-ethyl-4-fluoro-5-methylsulphonylbenzamide;
according to Claim 1, and their physiologically acceptable salts.

3. Process for the preparation of ortho-substituted benzoic acid derivatives of the formula I according to Claim 1, and of their salts, characterized in that
- a carboxylic acid of the formula I (Q = hydroxyl) is converted into an acylguanidine,
and/or in that
- a compound of the formula I is converted into one of its salts by treating with an acid or a base.

4. Process for the production of a pharmaceutical preparation, characterized in that a compound of the formula I according to Claim 1, in which Q is -N=C(NH₂)₂ and/or one of its physiologically acceptable salts is brought into a suitable administration form together with at least one solid, liquid or semi-liquid vehicle or excipient.

5. Pharmaceutical preparation, characterized in that it contains at least one compound of the formula I, according to Claim 1, in which Q is -N=C(NH₂)₂, and/or one of its physiologically acceptable salts.

6. Use of a compound of the formula I according to Claim 1, in which Q is -N=C(NH₂)₂, and/or one of its physiologically acceptable salts for the production of medicaments.

7. Use of compounds of the formula I according to Claim 1 in which Q is -N=C(NH₂)₂, for the production of medicaments for the treatment of arrhythmias, angina pectoris, infarcts and also for the preventive treatment of the indications mentioned.

## Revendications

1. Dérivés de l'acide benzoïque substitués en position ortho de formule I 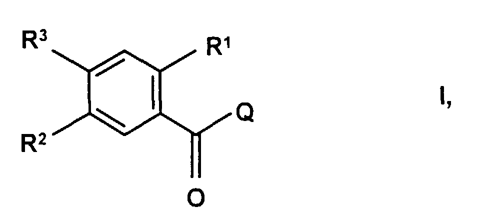 dans laquelle
R¹ représente A,
R² représente -SO₂-R⁶,
R³ représente CN ou un atome d'halogène,
Q représente -N=C(NH₂)₂,
R⁶ représente A,
A représente un groupe alkyle ayant 1 à 6 atomes de C, ainsi que leurs sels acceptables sur le plan pharmaceutique.

2. (a) le N-diaminométhylhne-2-méthyl-4-bromo-5-méthylsulfonylbenzamide ;
(b) le N-diaminométhylène-2-méthyl-4-chloro-5-méthylsulfonylbenzamide ;
(c) le N-diaminométhylène-2-méthyl-4-cyano-5-méthylsulfonylbenzamide ;
(d) le N-diaminométhylène-2-éthyl-4-bromo-5-méthylsulfonylbenzamide ;
(e) le N-diaminométhylène-2-éthyl-4-chloro-5-méthylsulfonylbenzamide ;
(f) le N-diaminométhylène-2-méthyl-4-fluoro-5-méthylsulfonylbenzamide ;
(g) le N-diaminométhylène-2-éthyl-4-fluoro-5-méthylsulfonylbenzamide ;
selon la revendication 1, ainsi que leurs sels inoffensifs sur le plan physiologique.

3. Procédé de préparation de dérivés de l'acide benzoïque substitués en position ortho de formule I selon la revendication 1, ainsi que de leurs sels, caractérisé en ce que l'on convertit
- un acide carboxylique de formule I (Q = groupe hydroxyle) en une acylguanidine,
et/ou que l'on convertit
- un composé de formule I en un de ses sels par traitement avec un acide ou une base.

4. Procédé de production d'une préparation pharmaceutique, caractérisé en ce que l'on transforme un composé de formule I selon la revendication 1, dans laquelle Q représente -N=C(NH₂)₂ et/ou un de ses sels inoffensifs sur le plan physiologique, conjointement avec au moins un véhicule ou adjuvant solide, semi-liquide ou liquide en une forme pharmaceutique appropriée.

5. Préparation pharmaceutique, caractérisée par une teneur en au moins un composé de formule I, selon la revendication 1, dans laquelle Q représente -N=C(NH₂)₂ et/ou un de ses sels inoffensifs sur le plan physiologique.

6. Utilisation d'un composé de formule 1 selon la revendication 1, dans laquelle Q représente -N=C(NH₂)₂ et/ou un de ses sels inoffensifs sur le plan physiologique afin de produire des médicaments.

7. Utilisation de composés de formule 1 selon la revendication 1, dans laquelle Q représente -N=C(NH₂)₂, afin de produire des médicaments pour traiter les arythmies, l'angine de poitrine, les infarctus ainsi que pour le traitement préventif des indications citées.
